# EUROPEAN PATENT SPECIFICATION

(11) **EP 3 878 785 B1**
(45) Date of publication and mention of the grant of the patent: **06.09.2023**
(21) Application number: 21160176.0
(22) Date of filing: 02.03.2021
(51) Int. Cl.: B65H 63/06, G01N 21/89, G01N 33/36, G01N 21/952

(54) **YARN MONITORING DEVICE AND YARN WINDING MACHINE**
GARNÜBERWACHUNGSVORRICHTUNG UND GARNWICKELMASCHINE
DISPOSITIF DE SURVEILLANCE DE FIL ET MACHINE DE BOBINAGE DE FIL

(30) Priority: 10.03.2020 JP 2020040991
(43) Date of publication of application: 15.09.2021
(73) Proprietor: Murata Machinery, Ltd., Kyoto-shi, Kyoto 601-8326 (JP)
(72) Inventor: Kawabata, Satoshi, Kyoto, 612-8686 (JP)
(74) Representative: Hoffmann Eitle

(56) References cited:
- EP-A1- 1 508 798
- EP-A1- 1 808 690
- JP-A- 2015 081 402
- JP-B2- 5 413 560
- US-A- 5 130 559

## Description

### TECHNICAL FIELD

The present invention relates to a yarn monitoring device and a yarn winding machine.

### BACKGROUND

As a technique related to a yarn monitoring device, a yarn clearer described in JP 5413560 B is known. The yarn clearer described in JP 5413560 B measures light (transmitted light) part of which has been blocked by spun yarn (yarn), thereby detecting the thickness of the spun yarn. The yarn clearer described in JP 5413560 B also measures reflected light reflected by the spun yarn, thereby detecting a foreign substance included in the spun yarn. JP 2015 081402 A discloses a yarn monitoring device according to the preamble of claim 1, and EP 1508798 A1 as well as EP 1808690 A1 relate to similar devices.

### SUMMARY

While there are various types of yarn defects, it is difficult for a yarn monitoring device as described above to distinguish types of such yarn defects, and it may be difficult to accurately discriminate the yarn defects.

In view of this, it is an object of the present invention to provide a yarn monitoring device and a yarn winding machine that can accurately discriminate yarn defects.

A yarn monitoring device according to the present invention is a yarn monitoring device configured to monitor yarn traveling, and includes a discrimination section configured to discriminate a type of a yarn defect of the yarn, based on a combination of thickness of the yarn and brightness of reflected light that has been reflected by the yarn when the yarn has been irradiated with light.

In this yarn monitoring device, based on the combination of the thickness of the yarn and the brightness of the reflected light reflected by the yarn, the type of the yarn defect is discriminated. By this configuration, a yarn defect that is difficult to be distinguished based on only either one of the thickness and the brightness can be distinguished, whereby the yarn defect can be accurately discriminated.

In the yarn monitoring device according to the present invention, the discrimination section may discriminate, as the yarn defect, a first yarn defect having warpage, tangle, or entanglement protruding in a direction intersecting a direction in which the yarn travels. It is found that the first yarn defect (hereinafter, also called "kink") having warpage, tangle, or entanglement protruding in a direction intersecting the traveling direction of the yarn can be discriminated by combining the thickness of the yarn and the brightness of the reflected light reflected by the yarn. Thus, in the present invention, a kink as the yarn defect can be accurately discriminated.

In the yarn monitoring device according to one aspect of the present invention, the discrimination section may discriminate, as the yarn defect, a second yarn defect being a nep into which a foreign substance is mixed. It is found that the second yarn defect (hereinafter, also called "foreign-substance mixed nep") being a nep into which a foreign substance is mixed can be discriminated by combining the thickness of the yarn and the brightness of the reflected light reflected by the yarn. Thus, in the present invention, a foreign-substance mixed nep as the yarn defect can be accurately discriminated.

In the yarn monitoring device according to the present invention, the discrimination section includes a first defect-discrimination-target identification section and a second defect-discrimination-target identification section. The first defect-discrimination-target identification section identifies, in a first variation signal for thickness, a first defect-discrimination-target portion for which a defect is to be assessed. The second defect-discrimination-target identification section identifies, in a second variation signal for brightness, a second defect-discrimination-target portion for which a defect is to be assessed. The discrimination section identifies the first defect-discrimination-target portion and the second defect-discrimination-target portion that correspond to each other in a longitudinal direction of the yarn, and discriminates the type of the yarn defect based on the thickness of the first defect-discrimination-target portion and the brightness of the second defect-discrimination-target portion that have been identified as portions corresponding to each other. By this configuration, discrimination of the yarn defect based on the combination of the thickness of the yarn and the brightness of the reflected light reflected by the yarn can be specifically performed.

In the yarn monitoring device according to the present invention, the discrimination section determines that a first yarn defect having warpage, tangle, or entanglement protruding in a direction intersecting a direction in which the yarn travels is present when the thickness of the first defect-discrimination-target portion is equal to or greater than a first thickness criterion value and the brightness of the second defect-discrimination-target portion is equal to or higher than a first brightness criterion value. When the yarn defect is a kink, it is found that a detected thickness increases to a certain thickness or greater and also a detected brightness increases to a certain brightness or higher because light is irregularly reflected by the kink. Thus, according to the present invention, a kink can be more accurately discriminated.

In the yarn monitoring device according to one aspect of the present invention, the second defect-discrimination-target portion may include a continuous portion in which the brightness is equal to or higher than a threshold in the yarn, the continuous portion coinciding with at least part of the first defect-discrimination-target portion. By this configuration, discrimination of the yarn defect based on the combination of the thickness of the yarn and the brightness of the reflected light reflected by the yarn can be specifically performed.

In the yarn monitoring device according to one aspect of the present invention, the discrimination section may determine that a second yarn defect being a nep into which a foreign substance is mixed is present when the thickness of the first defect-discrimination-target portion is equal to or greater than a second thickness criterion value and the brightness of the second defect-discrimination-target portion is equal to or lower than a second brightness criterion value. When the yarn defect is a foreign-substance mixed nep, it is found that the detected thickness increases to a certain thickness or greater and also the detected brightness decreases to a certain brightness or lower because light is absorbed by the foreign substance. Thus, according to the present invention, a foreign-substance mixed nep can be more accurately discriminated.

In the yarn monitoring device according to one aspect of the present invention, the second defect-discrimination-target portion may include a continuous portion in which the brightness is equal to or lower than a threshold in the yarn, the continuous portion coinciding with at least part of the first defect-discrimination-target portion. By this configuration, discrimination of the yarn defect based on the combination of the thickness of the yarn and the brightness of the reflected light reflected by the yarn can be specifically performed.

In the yarn monitoring device according to one aspect of the present invention, the discrimination section may determine the presence or absence of a nep into which no foreign substance is mixed, based on the length of a continuous portion in which the thickness is equal to or greater than a threshold in the yarn and the thickness of the continuous portion. By this configuration, the presence or absence of a nep into which no foreign substance is mixed (hereinafter, also simply called "nep") can be accurately determined.

In the yarn monitoring device according to one aspect of the present invention, the discrimination section may determine the presence or absence of mixing of a foreign substance, based on the length of a continuous portion in which the brightness is equal to or lower than a threshold in the yarn and the brightness of the continuous portion. By this configuration, the presence or absence of mixing of a foreign substance can be accurately determined.

The yarn monitoring device according to one aspect of the present invention may include: a light emitting element configured to emit light toward a traveling region in which the yarn travels; a first light receiving element disposed so as to receive light that has been emitted from the light emitting element and part of which has been blocked by the yarn; and a second light receiving element disposed so as to receive light that has been emitted from the light emitting element and has been reflected by the yarn, in which the thickness may be calculated based on the amount of light received by the first light receiving element, and the brightness may be calculated based on the amount of light received by the second light receiving element. By this configuration, the thickness of the yarn and the brightness of the reflected light reflected by the yarn can be specifically calculated.

In the yarn monitoring device according to one aspect of the present invention, the first defect-discrimination-target identification section may identify the first defect-discrimination-target portion based on the first variation signal and a first threshold, the second defect-discrimination-target identification section may identify the second defect-discrimination-target portion based on the second variation signal and a second threshold, and the discrimination section may identify the first defect-discrimination-target portion and the second defect-discrimination-target portion overlapping each other in the longitudinal direction of the yarn as the portions corresponding to each other. By this configuration, the first defect-discrimination-target portion and the second defect-discrimination-target portion can be accurately identified.

In the yarn monitoring device according to one aspect of the present invention, the discrimination section may identify a peak position of the first defect-discrimination-target portion, the discrimination section may identify a peak position of the second defect-discrimination-target portion, and the discrimination section may identify, as the portions corresponding to each other, the first defect-discrimination-target portion and the second defect-discrimination-target portion the peak positions of which fall within a predetermined range in the longitudinal direction. By this configuration, the first defect-discrimination-target portion and the second defect-discrimination-target portion can be accurately identified.

The yarn monitoring device according to one aspect of the present invention may further include a display section configured to display a discrimination result of the discrimination section with a scatter diagram axes of which represent the thickness and the brightness. By this configuration, the discrimination result of the discrimination section can be effectively displayed by using the scatter diagram.

In the yarn monitoring device according to one aspect of the present invention, the display section may display a first yarn defect having warpage, tangle, or entanglement protruding in a direction intersecting a direction in which the yarn travels so as to distinguish the first yarn defect from another yarn defect. In the yarn monitoring device according to the present invention, the display section may display a second yarn defect being a nep into which a foreign substance is mixed so as to distinguish the second yarn defect from another yarn defect.

A yarn winding machine according to one aspect of the present invention includes: the yarn monitoring device described above; a yarn feeder disposed so as to be able to feed the yarn; and a winding device configured to wind the yarn fed from the yarn feeder to form a package, in which the yarn monitoring device is disposed between the yarn feeder and the winding device.

In this yarn winding machine also, the yarn monitoring device described above is included, and thus the above-described effect, that is, the effect of being able to accurately discriminate the yarn defect can be obtained.

According to the present invention, the yarn monitoring device and the yarn winding machine that can accurately discriminate the yarn defect can be provided.

### BRIEF DESCRIPTION OF THE DRAWINGS

FIG. 1 is a front view of a spinning machine according to an embodiment.
FIG. 2 is a block diagram illustrating a configuration of a yarn monitoring device in the spinning machine in FIG. 1.
FIG. 3 is a cross-sectional view illustrating one example of physical components of a device body of the yarn monitoring device.
FIG. 4 is a graph for describing a method of calculating a defect thickness and a defect length.
FIG. 5 is a graph for describing determination of the presence or absence of a nep.
FIG. 6A is an enlarged view exemplifying a kink, FIG. 6B is an enlarged view exemplifying a kink, and FIG. 6C is an enlarged view exemplifying a kink.
FIG. 7 is an enlarged photographic diagram exemplifying a foreign-substance mixed nep.
FIG. 8A is a graph for describing discrimination of a kink and is a graph representing the thickness and the length of yarn, and FIG. 8B is a graph for describing discrimination of a kink and is a graph representing the brightness and the length of the yarn.
FIG. 9A is a graph for describing discrimination of a foreign-substance mixed nep and is a graph representing the thickness and the length of the yarn, and FIG. 9B is a graph for describing discrimination of the foreign-substance mixed nep and is a graph representing the brightness and the length of the yarn.
FIG. 10 is a graph illustrating a scatter diagram as an example of a discrimination result of a discrimination section.
FIG. 11 is a graph illustrating a scatter diagram as another example of the discrimination result of the discrimination section.
FIG. 12 is a graph illustrating a scatter diagram as still another example of the discrimination result of the discrimination section.
FIG. 13 is a front view of an automatic winder according to the embodiment.

### DETAILED DESCRIPTION

An embodiment will now be described in detail with reference to the drawings. In the drawings, like or equivalent elements are designated by like numerals, and duplicate description is omitted.

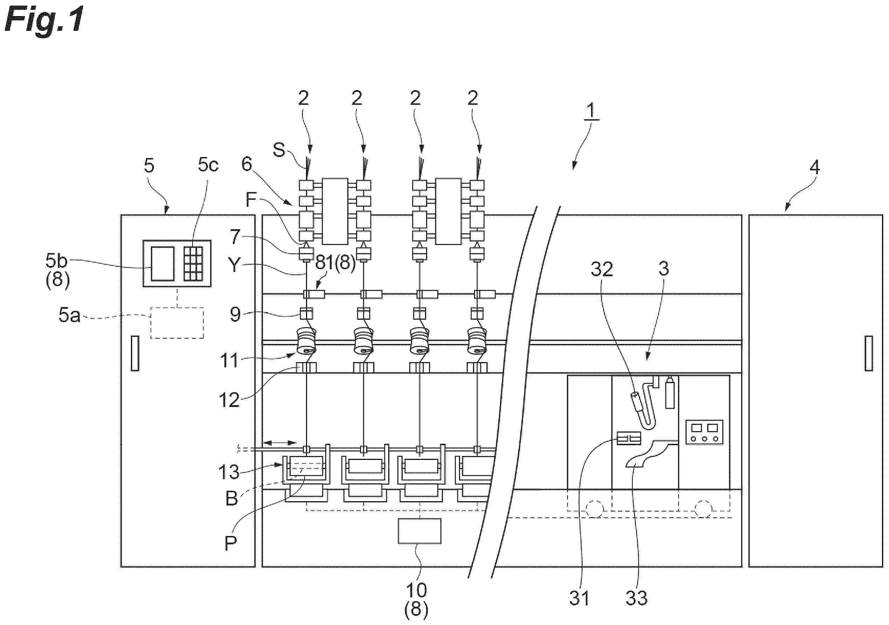

As illustrated in FIG. 1, a spinning machine (yarn winding machine) 1 includes a plurality of spinning units 2, a splicing carrier 3, a doffing carrier (not illustrated), a first end frame 4, and a second end frame 5. The spinning units 2 are aligned in a row. Each spinning unit 2 forms yarn Y and winds the yarn Y into a package P. When yarn Y has been cut or the yarn Y has broken for some reason in a certain spinning unit 2, the splicing carrier 3 performs splicing operation in the spinning unit 2. When a package P has been fully wound in a certain spinning unit 2, the doffing carrier doffs the package P, and supplies a new bobbin B to the spinning unit 2.

The first end frame 4 accommodates, for example, a collection device configured to collect fiber waste, yarn waste, and the like generated in the spinning unit 2. The second end frame 5 accommodates, for example, an air supply unit configured to adjust air pressure of compressed air (air) supplied to the respective units in the spinning machine 1 and supply the air to the respective units in the spinning machine 1 and a drive motor configured to supply power to the respective units in the spinning units 2. The second end frame 5 includes a machine control device 5a, a display 5b, and an input keys 5c. The machine control device 5a centrally manages and controls the respective units of the spinning machine 1. The display 5b can display, for example, information on at least one of settings and the states of the spinning units 2. An operator can make the settings of the spinning units 2 by performing appropriate operations with the input keys 5c.

Each spinning unit 2 includes, in the order from the upstream side in a direction in which yarn Y travels, a drafting device 6, an air-jet spinning device 7, a yarn monitoring device 8, a tension sensor 9, a yarn storage device 11, a waxing device 12, and a winding device 13. The unit controller 10 is provided for every predetermined number of spinning units 2, and controls operations of the spinning units 2. In each spinning unit 2, the drafting device 6 and the air-j et spinning device 7 function as a yarn feeder configured to feed yarn Y.

The drafting device 6 drafts a sliver (fiber band) S. The air-jet spinning device 7 forms yarn Y by twisting a fiber band F drafted by the drafting device 6 using swirling airflow. The yarn storage device 11 eliminates slack in yarn Y between the air-j et spinning device 7 and the winding device 13. The waxing device 12 applies wax to yarn Y between the yarn storage device 11 and the winding device 13. The winding device 13 winds yarn Y fed from the drafting device 6 and the air-jet spinning device 7 that function as a yarn feeder around a bobbin B to form a package P.

The yarn monitoring device 8 is a device configured to monitor yarn Y traveling between the air-jet spinning device 7 and the yarn storage device 11. The yarn monitoring device 8 detects the presence or absence of a yarn defect on the basis of information on the monitored yarn Y. When having detected a yarn defect, the yarn monitoring device 8 transmits a yarn defect detection signal to the unit controller 10. The tension sensor 9 measures the tension of traveling yarn Y between the air-jet spinning device 7 and the yarn storage device 11, and transmits a tension measurement signal to the unit controller 10. When the unit controller 10 determines that abnormality has occurred based on at least one of the yarn defect detection signal and the tension measurement signal, the yarn Y is cut in the spinning unit 2.

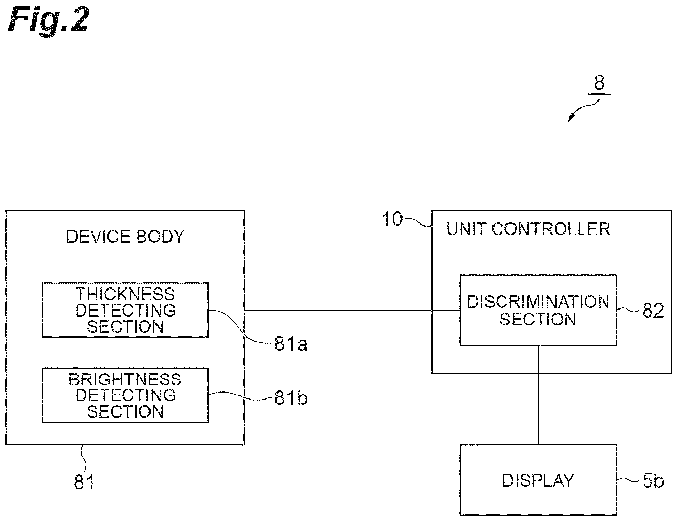

FIG. 2 is a block diagram illustrating a configuration of the yarn monitoring device 8. As illustrated in FIG. 2, the yarn monitoring device 8 includes a device body 81, a discrimination section 82, and the display 5b. The device body 81 includes a thickness detecting section 81a and a brightness detecting section 81b as functional components.

The thickness detecting section 81a is a section configured to detect the thickness of traveling yarn Y. The thickness can be expressed in terms of percentage when the reference yarn thickness that is a reference thickness of yarn Y is assumed to be zero. Detection of the thickness of yarn Y performed by the thickness detecting section 81a is detection of the apparent thickness of the yarn Y performed by an optical method in the present embodiment, but may be detection of the thickness of the yarn Y performed by a capacitive method. The brightness detecting section 81b is a section configured to detect the brightness of reflected light that has been reflected by yarn Y when the yarn Y has been irradiated with light (hereinafter, also simply called "brightness of yarn Y"). The brightness can be expressed in terms of percentage when the reference yarn brightness that is a reference brightness of yarn Y is assumed to be zero. For example, the brightness of a pitch dark state (when light is blocked) can be expressed as -100%. The thickness detecting section 81a and the brightness detecting section 81b constitute a detecting unit. The detecting unit is not limited to a particular one, and various types of known sensors may be used if the thickness and the brightness of yarn Y can be detected.

The device body 81 thus configured includes a detection module and a case configured to accommodate the detection module as physical components. The detection module includes a light emitting element, a first light receiving element, and a second light receiving element, and detects the amount of transmitted light and the amount of reflected light of light emitted toward yarn Y. In the detection module, light is emitted (projected) from the light emitting element toward yarn Y traveling between the air-jet spinning device 7 and the yarn storage device 11. In the detectionmodule, light (transmitted light) of the light, part of which has been blocked by the yarn Y, is received by the first light receiving element. In the detection module, reflected light of the light, which has been reflected by the yarn Y, is received by the second light receiving element. In the detection module, a signal indicating the amount of received light (the amount of transmitted light) of the transmitted light received by the first light receiving element is output as a signal for the thickness of the yarn Y to the discrimination section 82. In the detection module, a signal indicating the amount of received light (the amount of reflected light) of the reflected light received by the second light receiving element is output as a signal for the brightness of the light reflected by the yarn Y to the discrimination section 82.

The light emitting element, the first light receiving element, and the second light receiving element may be disposed in the same position in the yarn traveling direction. By this configuration, the amount of transmitted light and the amount of reflected light related to the same portion of yarn Y can be acquired at the same timing. The first light receiving element and the second light receiving element may be disposed in positions different in the yarn traveling direction. Even in this configuration, the amount of transmitted light and the amount of reflected light related to the same portion of the yarn Y can be acquired at the same timing. The first light receiving element and the second light receiving element may include light emitting elements individually. Specifically, as the light emitting elements, a first light emitting element and a second light emitting element may be included. The first light receiving element receives light (transmitted light) that has been emitted by the first light emitting element and part of which has been blocked by the yarn Y. The second light receiving element receives reflected light that has been emitted from the second light emitting element and has been reflected by the yarn Y. The first light emitting element and the second light emitting element may be disposed in the same position in the yarn traveling direction, or may be disposed in different positions therein. The first light emitting element and the first light receiving element may be disposed in the same position in the yarn traveling direction, the second light emitting element and the second light receiving element may be disposed in the same position in the yarn traveling direction, and also the position of the first light emitting element and the first light receiving element in the yarn traveling direction may be different from the position of the second light emitting element and the second light receiving element in the yarn traveling direction. Even in this configuration, based on the yarn traveling speed and the separation distance between the first light receiving element and the second light receiving element in the yarn traveling direction, the amount of transmitted light and the amount of reflected light related to the same portion of the yarn Y can be associated with each other.

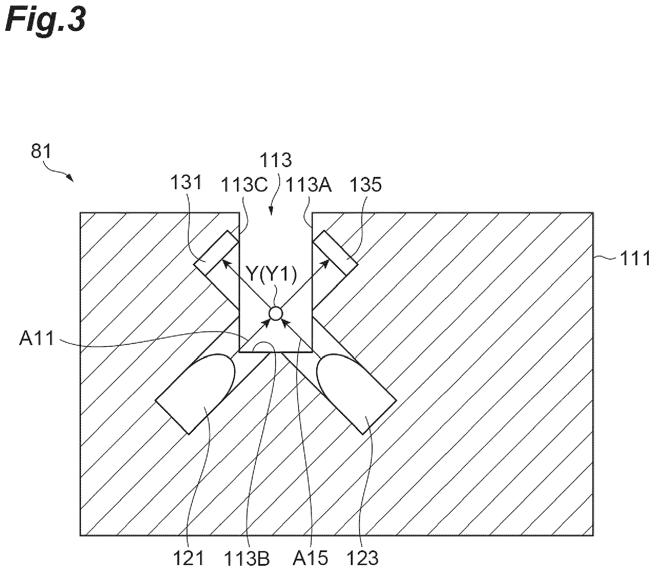

One example of physical components of the device body 81 will be described with reference to FIG. 3. As illustrated in FIG. 3, the device body 81 includes a holder 111, a first light projector (light emitting element) 121, a second light projector (light emitting element) 123, a first light receiver (first light receiving element) 131, and a second light receiver (second light receiving element) 135.

The holder 111 is configured as a resin housing, to which the first light projector 121, the second light projector 123, the first light receiver 131, and the second light receiver 135 are mounted. In the holder 111, a yarn passage 113 as a detection space including a portion serving as a yarn path along which yarn Y travels is formed along the traveling direction of the yarn Y. This yarn passage 113 has a shape that is open to the front side of the device.

The yarn passage 113 has a first surface 113A, a second surface 113B, and a third surface 113C. The first surface 113A and the third surface 113C are side wall surfaces on the right and left of the yarn passage 113. The second surface 113B is a deep wall surface of the yarn passage 113.

The first light projector 121 and the second light projector 123 project light to the yarn passage 113. The first light projector 121 and the second light projector 123 are, for example, bullet-shaped yellow LEDs having an outer surface made of resin the shape of which is formed in a bullet shape. Thus, light emitted from the first light projector 121 and light emitted from the second light projector 123 are each emitted in one direction as each of the optical axis directions A11, A15. Light emitting operations of the first light projector 121 and the second light projector 123 are controlled by the discrimination section 82.

The first light receiver 131 and the second light receiver 135 receive light from the first light projector 121 and the second light projector 123, respectively. Each of the first light receiver 131 and the second light receiver 135 is a photodiode, which converts the intensity of received light into an electrical signal and transmits the electrical signal to the discrimination section 82.

The first light projector 121 and the second light projector 123 are disposed so as to emit light from different angles onto yarn Y traveling through the yarn passage 113. The first light projector 121 and the second light projector 123 are controlled by the discrimination section 82 such that either one of them emits light alternately. By this configuration, the yarn Y can be irradiated with light from two different directions. The second yarn monitoring device 101 having two light projectors thus configured can measure the yarn Y from multiple angles, thereby being able to improve the accuracy of detecting a foreign substance and the yarn thickness.

The first light receiver 131 is disposed on the side opposite to the second light projector 123 with a yarn path Y1 of the yarn Y interposed therebetween. Thus, the first light receiver 131 receives transmitted light of light that has been emitted from the second light projector 123 onto the yarn Y. The second light receiver 135 on the other side is disposed on the side opposite to the first light projector 121 with the yarn path Y1 of the yarn Y interposed therebetween. Thus, the second light receiver 135 receives transmitted light of light that has been emitted from the first light projector 121 onto the yarn Y. Each light receiver configured to receive transmitted light is disposed in a position opposing to the corresponding light projector (position where light from the light projector directly strikes) with the yarn path Y1 interposed therebetween.

The first light receiver 131 is disposed such that light from the first light projector 121 does not directly strike thereon, and is disposed so as to be able to receive reflected light of light that has been emitted onto the yarn Y from the first light projector 121. The second light receiver 135 is disposed such that light from the second light projector 123 does not directly strike thereon, and is disposed so as to be able to receive reflected light of light that has been emitted onto the yarn Y from the second light projector 123. Each light receiver configured to receive reflected light is disposed in a position where light from the corresponding light projector does not directly strike.

In the above-described configuration, when the first light projector 121 emits light, the first light receiver 131 receives reflected light and the second light receiver 135 receives transmitted light. When the second light projector 123 emits light, the first light receiver 131 receives transmitted light and the second light receiver 135 receives reflected light.

Because the above-described transmitted light is light received by the first light receiver 131 or the second light receiver 135 while being blocked by the yarn Y, when the thickness of the yarn Y varies, the intensity (the amount of received light) of the transmitted light varies accordingly. Thus, by monitoring variations of the transmitted light received by the first light receiver 131 or the second light receiver 135 in the discrimination section 82, thickness nonuniformity of yarn Y (yarn irregularity) can be detected.

Because the above-described reflected light is light that have been reflected at a surface of the yarn Y and received by the first light receiver 131 or the second light receiver 135, the intensity (the amount of received light) of the reflected light also varies depending on the reflectance (color) of the surface of the yarn Y. Thus, by detecting variations of the reflected light received by the first light receiver 131 or the second light receiver 135 in the discrimination section 82, a foreign substance such as colored yarn included in the yarn Y can be detected.

Referring back to FIG. 2, the discrimination section 82 is incorporated in the unit controller 10. The discrimination section 82 discriminates a yarn defect of yarn Y on the basis of a first variation signal for the thickness of the yarn Y and a second variation signal for the brightness of the yarn Y. The discrimination section 82 discriminates the type of the yarn defect of the yarn Y on the basis of the first variation signal and the second variation signal. The yarn defect may be also called "defect", "yarn fault", and "fault", for example. Discrimination of a yarn defect may be also called "determination of a yarn defect", "detection of a yarn defect", and "assessment of a yarn defect". The discrimination section 82 has a plurality of channels, and can detect the presence or absence of different types of yarn defects with the respective channels.

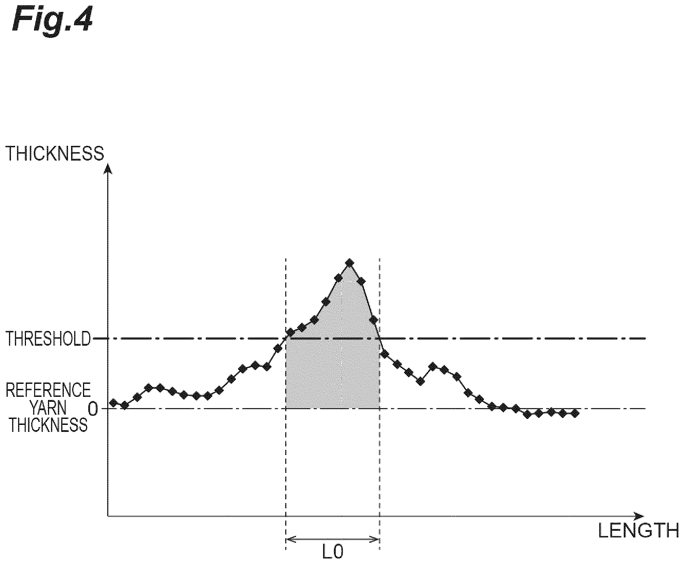

The discrimination section 82 determines the presence or absence of a nep on the basis of a defect thickness and a defect length (first defect length) in a first channel. Specifically, the discrimination section 82 calculates the defect thickness and the defect length. An example of a method of calculating the defect thickness and the defect length will be described below first with reference to FIG. 4.

The discrimination section 82 samples a detection signal indicating the amount of transmitted light (a yarn nonuniformity signal, the first variation signal) for every predetermined length (e.g., 1 mm), and then takes the moving average thereof for an appropriate length (e.g., 2 mm), and calculates the defect thickness and a defect length L0 according to the followings (1) to (3). In the graph of FIG. 4, the X-axis represents length and the Y-axis represents thickness. Herein, the defect length corresponds to the length of a continuous portion in which the thickness is equal to or greater than a threshold in the yarn Y. The defect thickness corresponds to the thickness of the continuous portion. The continuous portion in which the thickness is equal to or greater than the threshold is a first defect-discrimination-target portion, which is a portion corresponding to a yarn defect that needs to be cut or a yarn defect that is allowed to remain in the yarn Y without being cut.
(1) Calculate the length of a continuous portion in which the thickness is equal to or greater than the threshold as the defect length L0. For example, the threshold is the peak thickness of the continuous portion × a (e.g., a is 0.5) (50%).
(2) Determine a defect area. For example, the defect area can be determined by the integral of thickness of a portion corresponding to the defect length L0 (gray portion in FIG. 4).
(3) Determine the defect thickness as "the defect area / the defect length L0".

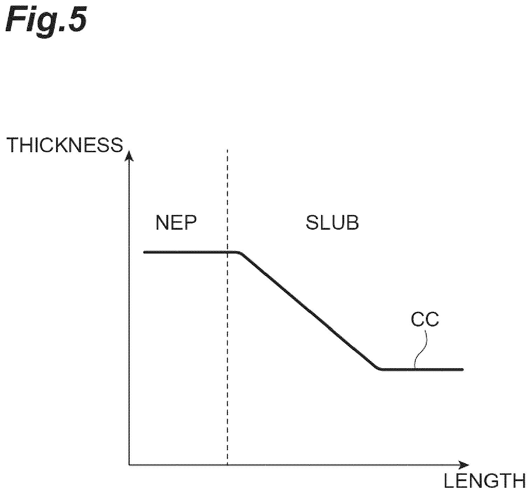

Subsequently, the discrimination section 82 compares the defect thickness and the defect length thus calculated with an discrimination threshold (what is called a clearing curve) CC as illustrated in FIG. 5, thereby determining the presence or absence of a nep. For example, if the defect length is equal to or smaller than a predetermined value (e.g., 1 cm) and the defect thickness is equal to or greater than the discrimination threshold, the discrimination section 82 determines that this continuous portion is a nep. If the defect length is greater than the predetermined value and the defect thickness is equal to or greater than the discrimination threshold, the discrimination section 82 determines that this yarn defect is a slub. The nep is a thick yarn defect having a thickness of 1 centimeter or smaller, for example. The slub is a thick yarn defect having a thickness smaller than several centimeters, for example.

The discrimination section 82 determines the presence or absence of mixing of a foreign substance on the basis of a defect brightness and a defect length (second defect length) in a second channel. Specifically, the discrimination section 82 calculates the defect brightness and the defect length. The defect brightness and the length thereof can be calculated by the same method as the above-described method of calculating the defect thickness and the length thereof. Subsequently, the discrimination section 82 compares the defect brightness and the defect length thus calculated with an discrimination threshold, thereby determining the presence or absence of mixing of a foreign substance. For example, if the defect brightness is equal to or higher than the discrimination threshold, the discrimination section 82 determines that this yarn defect is mixing of a foreign substance. Herein, the defect length in the second channel corresponds to the length of a continuous portion in which the brightness is equal to or higher than the threshold in the yarn Y. The defect brightness corresponds to the brightness of this continuous portion. The continuous portion in which the brightness is equal to or higher than the threshold is a second defect-discrimination-target portion, which is a portion corresponding to a yarn defect that needs to be cut or a yarn defect that is allowed to remain in the yarn Y without being cut.

The discrimination section 82 according to the present embodiment discriminates a yarn defect of yarn Y on the basis of a combination of the thickness and the brightness of the yarn Y in a third channel. The discrimination section 82 determines the presence or absence of a kink in the yarn Y, the presence or absence of a foreign-substance mixed nep in the yarn Y, and whether the yarn Y is normal yarn on the basis of the thickness and the brightness of the yarn Y in the third channel.

The discrimination section 82 includes a first defect-discrimination-target identification section and a second defect-discrimination-target identification section. The first defect-discrimination-target identification section identifies, in a first variation signal for thickness, a first defect-discrimination-target portion for which a defect is to be assessed. The second defect-discrimination-target identification section identifies, in a second variation signal for brightness, a second defect-discrimination-target portion for which a defect is to be assessed. The discrimination section 82 identifies the first defect-discrimination-target portion and the second defect-discrimination-target portion that correspond to each other in the longitudinal direction of the yarn Y, and discriminates the type of the yarn defect on the basis of the thickness of the first defect-discrimination-target portion and the brightness of the second defect-discrimination-target portion that have been identified as portions corresponding to each other. The first defect-discrimination-target identification section identifies the first defect-discrimination-target portion based on the first variation signal and a first threshold, the second defect-discrimination-target identification section identifies the second defect-discrimination-target portion based on the second variation signal and a second threshold. The discrimination section identifies the first defect-discrimination-target portion and the second defect-discrimination-target portion overlapping each other in the longitudinal direction of the yarn as the portions corresponding to each other. The discrimination section identifies a peak position of the first defect-discrimination-target portion, and identifies a peak position of the second defect-discrimination-target portion. The discrimination section identifies, as the portions corresponding to each other, the first defect-discrimination-target portion and the second defect-discrimination-target portion the peak positions of which fall within a predetermined range in the longitudinal direction.

Specifically, the first defect-discrimination-target identification section identifies, as the first defect-discrimination-targetportion, a portion that exceeds the first threshold in the first variation signal. The discrimination section 82 identifies the peak position (first peak position) of the first defect-discrimination-target portion. The second defect-discrimination-target identification section identifies, as the second defect-discrimination-target portion, a portion that exceeds the second threshold in the second variation signal. The discrimination section 82 identifies the peak position (second peak position) of a candidate for the second defect-discrimination-target portion. The discrimination section 82 identifies, as the second defect-discrimination-target portion corresponding to the first defect-discrimination-target portion, a portion in which the second peak position is present within a predetermined range of (within 1 cm, for example, both before and after) the first peak position in the longitudinal direction.

The discrimination section 82 calculates the length and the thickness of the first defect-discrimination-target portion. The discrimination section 82 calculates the length from a portion exceeding the first threshold. The discrimination section 82 calculates the thickness based on the area of a portion exceeding a reference yarn thickness (first reference value) that is different from the first threshold of the first defect-discrimination-target portion. The discrimination section 82 calculates the length and the brightness of the second defect-discrimination-target portion (that has been identified as a portion corresponding to the first defect-discrimination-target portion). The discrimination section 82 calculates the length from a portion exceeding the second threshold. The discrimination section 82 calculates the brightness based on the area of a portion exceeding a reference yarn brightness (second reference value) that is different from the second threshold of the second defect-discrimination-target portion. The discrimination section 82 discriminates the type of a yarn defect of the yarn Y on the basis of "the length and the thickness" and "the length and the brightness" that have been calculated. The first threshold and the reference yarn thickness (first reference value) are usually set to be different. This is because, if the first threshold and the reference yarn thickness (first reference value) are the same, too small variations are detected excessively, which causes processing load to increase excessively. However, under circumstances where the processing load can be ignored, the first threshold and the reference yarn thickness (first reference value) may be set the same. This applies to the relation between the second threshold and the reference yarn brightness (second reference value).

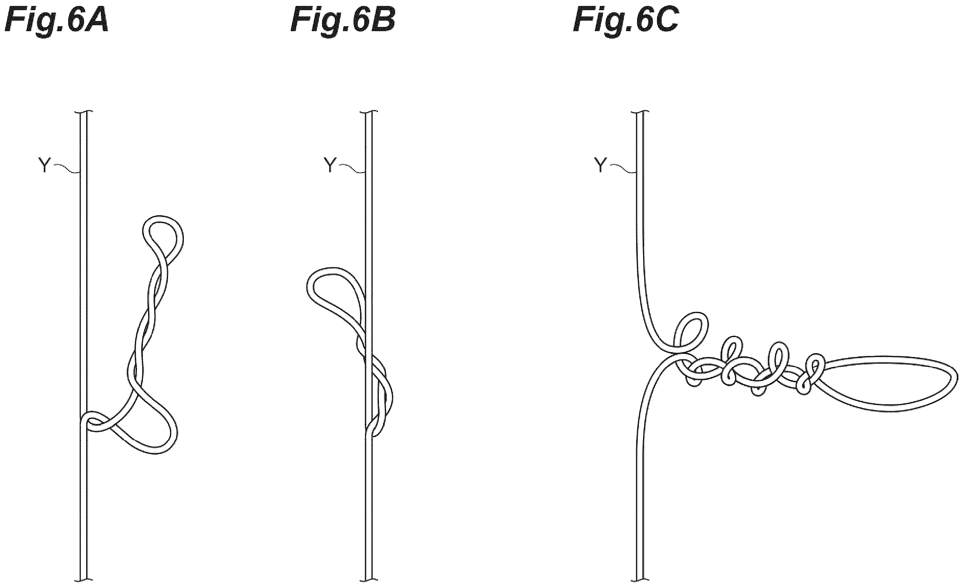

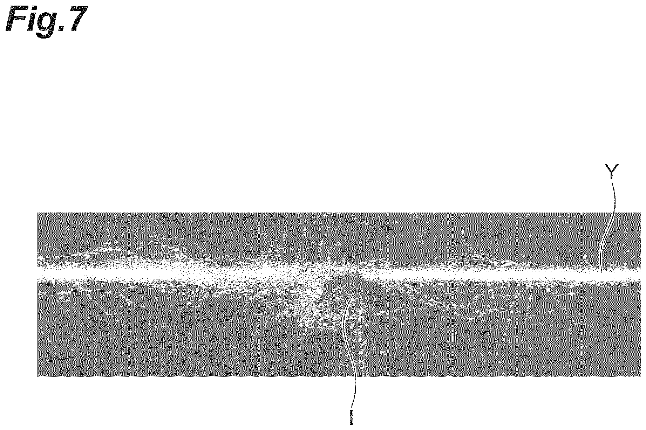

The following describes in detail one example in which, the presence or absence of a kink in yarn Y, the presence or absence of a foreign-substance mixed nep in the yarn Y, and whether the yarn Y is normal yarn are determined based on a combination of the thickness and the brightness of the yarn Y. For example, as illustrated in FIG. 6A, FIG. 6B, and FIG. 6C, a kink is a yarn defect having warpage, tangle, or entanglement protruding in a direction intersecting the traveling direction of the yarn Y. The kink includes snarling and sloughing. For example, as illustrated in FIG. 7, the foreign-substance mixed nep is a nep into which a foreign substance I such as large pepper trash is mixed. The kink corresponds to the first yarn defect, and the foreign-substance mixed nep corresponds to the second yarn defect.

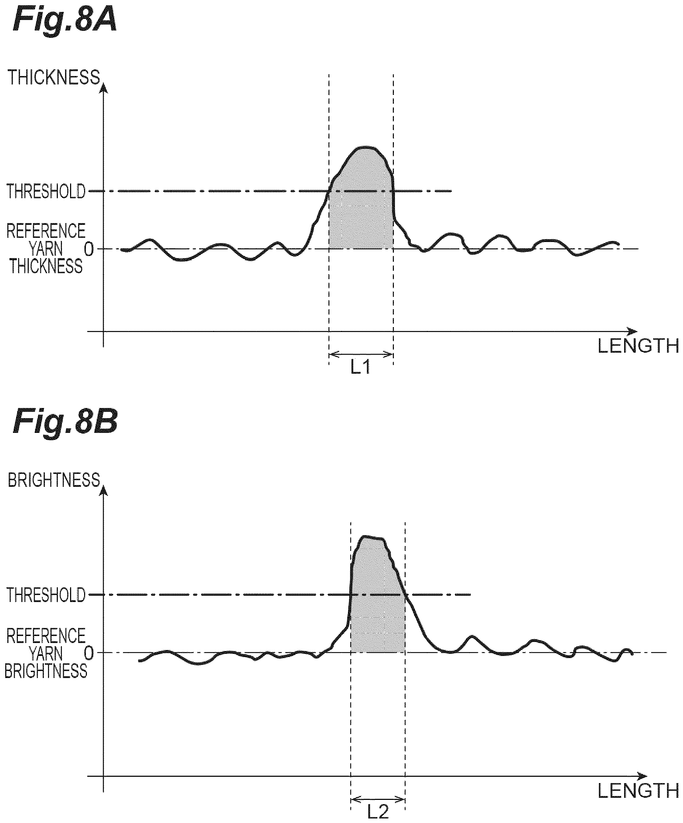

The discrimination section 82 determines the presence or absence of a kink in yarn Y on the basis of the thickness (defect thickness) of a continuous portion of the yarn Y that has been extracted as the first defect-discrimination-target portion and the brightness (defect brightness) of a continuous portion of the yarn Y that has been extracted as the second defect-discrimination-target portion at the same timing. Specifically, the discrimination section 82 appropriately samples a first variation signal indicating the thickness of the yarn Y and takes the moving average thereof, thereby obtaining a first variation signal for the thickness and the length of the yarn Y as illustrated in FIG. 8A. Using the first variation signal for the thickness and the length of the yarn Y, the discrimination section 82 calculates the thickness of the first defect-discrimination-target portion of the yarn Y.

The first defect-discrimination-target portion is a portion in which the thickness in the yarn Y is calculated and whether it is a yarn defect is to be determined. The first defect-discrimination-target portion herein is a continuous portion in which the thickness is equal to or greater than the threshold in the yarn Y as described above. The thickness of the first defect-discrimination-target portion can be determined by calculating "the area of first defect-discrimination-target portion" / "the length L1 of the first defect-discrimination-target portion" on the graph for the thickness and the length of the yarn Y. For example, the area of the first defect-discrimination-target portion can be determined from the integral (gray portion in FIG. 8A) of the thickness in the first defect-discrimination-target portion. The threshold for the first variation signal corresponds to the first threshold.

Furthermore, the discrimination section 82 appropriately samples a second variation signal indicating the brightness of the yarn Y and takes the moving average thereof, thereby obtaining a second variation signal for the brightness and the length of the yarn Y as illustrated in FIG. 8B. Using the second variation signal for the brightness and the length of the yarn Y, the discrimination section 82 calculates the brightness of the second defect-discrimination-target portion of the yarn Y.

The second defect-discrimination-target portion is a portion corresponding to the first defect-discrimination-target portion in the yarn Y, and is a portion for which the brightness is calculated and whether it is a yarn defect is to be determined. The second defect-discrimination-target portion herein is a continuous portion in which the brightness is equal to or higher than a threshold (peak brightness × 0.5) in the yarn Y. Because the thickness and the brightness of the yarn Y are obtained simultaneously by measuring the same portion of the yarn Y, the first defect-discrimination-target portion and the second defect-discrimination-target portion include the same continuous portion of the yarn Y. In other words, the second defect-discrimination-target portion includes a continuous portion that coincides with at least part of the first defect-discrimination-target portion. The brightness of the second defect-discrimination-target portion can be determined by calculating "the area of the second defect-discrimination-target portion" / "the length L2 of the second defect-discrimination-target portion" on the graph for the brightness and the length of the yarn Y. For example, the area of the second defect-discrimination-target portion can be determined from the integral (gray portion in FIG. 8B) of the brightness of the second defect-discrimination-target portion. The threshold for the second variation signal corresponds to the second threshold.

If the thickness of the first defect-discrimination-target portion is equal to or greater than a first thickness criterion value and the brightness of the second defect-discrimination-target portion is equal to or higher than a first brightness criterion value, the discrimination section 82 determines that a kink is present in the portions (the first defect-discrimination-target portion and the second defect-discrimination-target portion). The first thickness criterion value and the first brightness criterion value are predetermined thresholds for assessing a kink. The first thickness criterion value and the first brightness criterion value may be fixed values, or may be variable values. The first thickness criterion value and the first brightness criterion value are not limited to particular values, and can be set or changed appropriately by a user. The first brightness criterion value is a positive (plus) value. Even though the thickness of the first defect-discrimination-target portion is equal to or greater than the first thickness criterion value, if the brightness of the second defect-discrimination-target portion is higher than the reference yarn brightness and is lower than the first brightness criterion value, the discrimination section 82 determines that these portions fall under normal yarn.

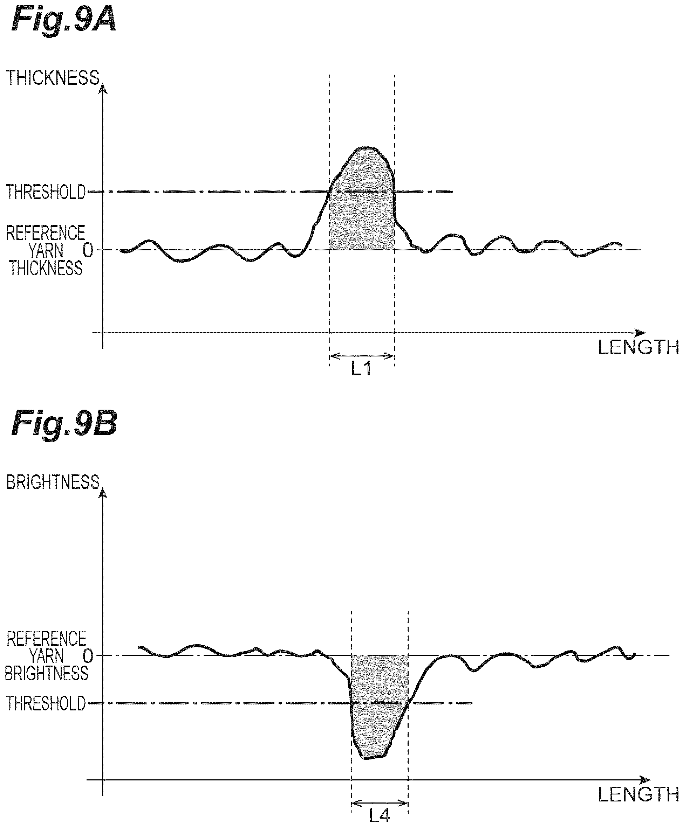

The discrimination section 82 determines the presence or absence of a foreign-substance mixed nep in yarn Y on the basis of the thickness of the first defect-discrimination-target portion of the yarn Y and the brightness of the second defect-discrimination-target portion of the yarn Y. Specifically, in the same manner as in the above-described discrimination of a kink, the discrimination section 82 calculates the thickness of the first defect-discrimination-target portion of the yarn Y, using the first variation signal for the thickness and the length of the yarn Y as illustrated in FIG. 9A. Furthermore, in the same manner as in the above-described discrimination of a kink, the discrimination section 82 calculates the brightness of the second defect-discrimination-target portion of the yarn Y, using the second variation signal for the brightness and the length of the yarn Y as illustrated in FIG. 9B. The second defect-discrimination-target portion is a portion of the yarn Y corresponding to the first defect-discrimination-target portion, which is a portion in which the brightness is to be monitored. The second defect-discrimination-target portion herein is a continuous portion in which the brightness is equal to or lower than a threshold (peak brightness × 0.5) in the yarn Y. The brightness of the second defect-discrimination-target portion can be determined by calculating "the area of the second defect-discrimination-target portion" / "the length L4 of the second defect-discrimination-target portion" on the graph for the brightness and the length of the yarn Y. For example, the area of the second defect-discrimination-target portion can be determined from the integral (gray portion in FIG. 9B) of the brightness of the second defect-discrimination-target portion.

If the thickness of the first defect-discrimination-target portion is equal to or greater than a second thickness criterion value and the brightness of the second defect-discrimination-target portion is equal to or lower than a second brightness criterion value, the discrimination section 82 determines that a foreign-substance mixed nep is present in the portions (the first defect-discrimination-target portion and the second defect-discrimination-target portion). The second thickness criterion value and the second brightness criterion value are predetermined thresholds for assessing a foreign-substance mixed nep. The second thickness criterion value and the second brightness criterion value may be fixed values, or may be variable values. The second thickness criterion value and the second brightness criterion value are not limited to particular values, and can be set or changed appropriately by the user. The second thickness criterion value and the first thickness criterion value may be the same, or may be different. The second brightness criterion value is a negative (minus) value. Even though the thickness of the first defect-discrimination-target portion is equal to or greater than the second thickness criterion value, if the brightness of the second defect-discrimination-target portion is higher than the second brightness criterion value and lower than the reference yarn brightness, the discrimination section 82 determines that these portions fall under normal yarn.

Herein, in the third channel, the discrimination section 82 may determine the presence or absence of a nep in the yarn Y and the presence or absence of mixing of a foreign substance therein. For example, in the third channel, if the thickness of the first defect-discrimination-target portion is equal to or greater than a third thickness criterion value and the brightness of the second defect-discrimination-target portion is lower than the first brightness criterion value and higher than the second brightness criterion value, the discrimination section 82 may determine that a nep is present in these portions. For example, in the third channel, if the thickness of the first defect-discrimination-target portion is smaller than the second thickness criterion value and the brightness of the second defect-discrimination-target portion is equal to or lower than the second brightness criterion value, the discrimination section 82 may determine that mixing of a foreign substance is present in these portions.

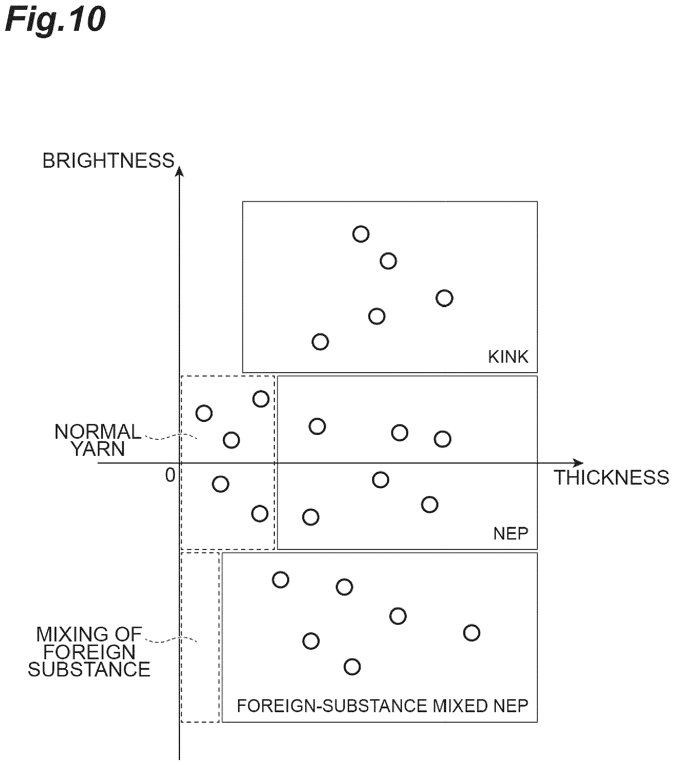

Referring back to FIG. 2, the display 5b displays a discrimination result of the discrimination section 82 with a scatter diagram the axes of which represent the thickness of yarn Y and the brightness of the yarn Y. For example, as illustrated in FIG. 10, in coordinates with the X-axis representing the thickness and the Y-axis representing the brightness, a graph in which a plurality of data points (circles in FIG. 10) are plotted for each yarn defect is displayed on the display 5b. In the graph, the area is divided into respective areas for a kink, a nep, a foreign-substance mixed nep, mixing of a foreign substance, and normal yarn, and yarn defects of the yarn Y can be easily grasped based on the data plots and their respective areas. In FIG. 10, the first thickness criterion value, the second thickness criterion value, the first brightness criterion value, and the second brightness criterion value vary in the longitudinal direction (direction orthogonal to the plane of FIG. 10) .

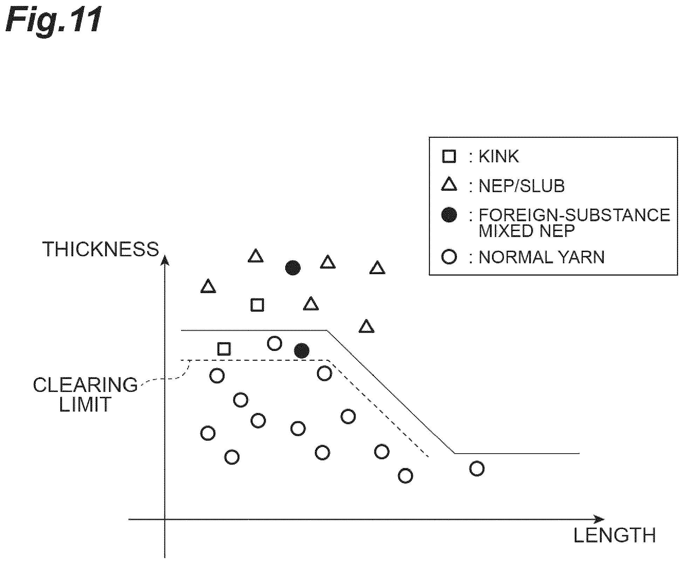

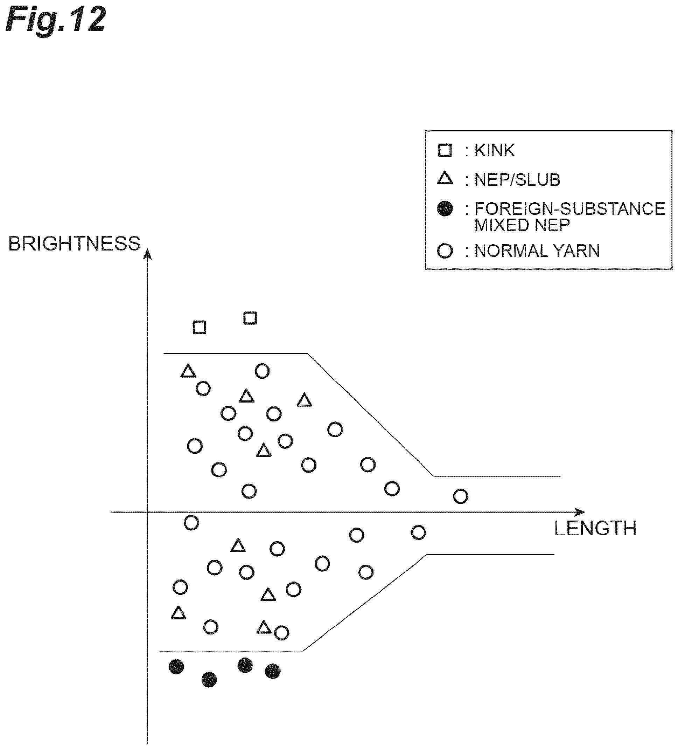

The display 5b can also display a scatter diagram in which the Y-axis and the X-axis represent the thickness and the length, respectively, as illustrated in FIG. 11, for example. The display 5b can also display a scatter diagram in which the Y-axis and the X-axis represent the brightness and the length, respectively, as illustrated in FIG. 12, for example. The display 5b can also change displays between the scatter diagram illustrated in FIG. 11 and the scatter diagram illustrated in FIG. 12.

In each spinning unit 2 of the spinning machine 1 described above, when yarn Y is being spun, the yarn Y is monitored by the yarn monitoring device 8. The discrimination section 82 of the yarn monitoring device 8 determines the presence or absence of a nep in the first channel. When it has been determined that a nep is present, the yarn Y is cut such that the nep is removed. When it has been determined that a nep is absent, the discrimination section 82 of the yarn monitoring device 8 determines the presence or absence of mixing of a foreign substance in the second channel.

When it has been determined that mixing of a foreign substance is present, the yarn Y is cut such that the yarn defect of this mixing of a foreign substance is removed. When it has been determined that mixing of a foreign substance is absent, the discrimination section 82 of the yarn monitoring device 8 determines the presence or absence of a kink and the presence or absence of a foreign-substance mixed nep in the third channel. When it has been determined that a kink or a foreign-substance mixed nep is present, the yarn Y is cut such that the kink or the foreign-substance mixed nep is removed. When it has been determined that a kink and a foreign-substance mixed nep are absent, the discrimination section 82 of the yarn monitoring device 8 determines that the yarn Y is normal yarn, and spinning of the yarn Y is continued normally. After the yarn Y has been cut, the splicing carrier 3 performs splicing operation in the spinning unit 2.

As described in the foregoing, in the yarn monitoring device 8, the type of a yarn defect is discriminated based on a combination of the thickness of yarn Y and the brightness of the yarn Y. By this configuration, a yarn defect that is difficult to be distinguished only based on either one of the thickness of yarn Y and the brightness of the yarn Y can be distinguished, whereby the yarn defect can be accurately discriminated.

In the yarn monitoring device 8, the discrimination section 82 discriminates a kink on the basis of a combination of the thickness of the yarn Y and the brightness of light reflected by the yarn Y in the third channel. In the case of a common nep or slub, the amount of the reflected light does not vary so much. However, in the case of a kink, emitted light is irregularly reflected, and thus the amount of the reflected light increases. It is found that, by utilizing this characteristic and combining the thickness of yarn Y and the brightness of the yarn Y, the kink can be discriminated. In other words, in the yarn monitoring device 8, the kink can be accurately discriminated as a yarn defect.

In the yarn monitoring device 8, the discrimination section 82 discriminates a foreign-substance mixed nep on the basis of a combination of the thickness of yarn Y and the brightness of the yarn Y in the third channel. A foreign substance I such as pepper trash is colored, and thus absorbs reflected light, which causes the brightness of the yarn Y to change. It is found that, by utilizing this characteristic and combining the thickness of the yarn Y and the brightness of the yarn Y, the foreign-substance mixed nep can be discriminated. In other words, in the yarn monitoring device 8, the foreign-substance mixed nep can be accurately discriminated as a yarn defect.

In the yarn monitoring device 8, the discrimination section 82 includes the first defect-discrimination-target identification section and the second defect-discrimination-target identification section. The first defect-discrimination-target identification section identifies, in a first variation signal for thickness, a first defect-discrimination-target portion for which a defect is to be assessed. The second defect-discrimination-target identification section identifies, in a second variation signal for brightness, a second defect-discrimination-target portion for which a defect is to be assessed. The discrimination section 82 identifies the first defect-discrimination-target portion and the second defect-discrimination-target portion that correspond to each other in the longitudinal direction of the yarn Y, and discriminates the type of the yarn defect based on the thickness of the first defect-discrimination-target portion and the brightness of the second defect-discrimination-target portion that have been identified as portions corresponding to each other. By this configuration, discrimination of the yarn defect based on the combination of the thickness of the yarn Y and the brightness of the yarn Y can be specifically performed.

In the yarnmonitoringdevice 8, the discrimination section 82 determines that a kink is present if the thickness of the first defect-discrimination-target portion is equal to or greater than a first thickness criterion value and the brightness of the second defect-discrimination-target portion is equal to or higher than a first brightness criterion value in the third channel. When the yarn defect is a kink, it is found that the thickness of the yarn Y increases to a certain thickness or greater and also the brightness of the yarn Y increases to a certain brightness or higher. Thus, with the yarn monitoring device 8, a kink can be more accurately discriminated.

In the yarn monitoring device 8, when the presence or absence of a kink is determined, the first defect-discrimination-target portion is a continuous portion in which the thickness of the yarn Y is equal to or greater than a threshold (e.g., the continuous portion having the length L1 in FIG. 8A), and the second defect-discrimination-target portion includes a continuous portion in which the brightness of the yarn Y is equal to or higher than a threshold (e.g., the continuous portion having the length L2 in FIG. 8B) and that coincides with at least part of the first defect-discrimination-target portion. In the yarn monitoring device 8, when the presence or absence of a foreign-substance mixed nep is determined, the first defect-discrimination-target portion is a continuous portion in which the thickness of the yarn Y is equal to or greater than a threshold (e.g., the continuous portion having the length L1 in FIG. 9A), and the second defect-discrimination-target portion includes a continuous portion in which the brightness of the yarn Y is equal to or lower than a threshold (e.g., the continuous portion having the length L4 in FIG. 9B) and that coincides with at least part of the first defect-discrimination-target portion. By this configuration, discrimination of the yarn defect based on the combination of the thickness of the yarn Y and the brightness of the yarn Y can be specifically performed.

In the yarn monitoring device 8, the discrimination section 82 determines that a foreign-substance mixed nep is present if the thickness of the first defect-discrimination-target portion is equal to or greater than a second thickness criterion value, and the brightness of the second defect-discrimination-target portion is equal to or lower than a second brightness criterion value in the third channel. When the yarn defect is a foreign-substance mixed nep, it is found that the detected thickness increases to a certain thickness or greater and also the detected brightness decreases to a certain brightness or lower because light is absorbed by the foreign substance. Thus, with this yarn monitoring device 8, a foreign-substance mixed nep can be more accurately discriminated.

In the yarn monitoring device 8, the discrimination section 82 determines the presence or absence of a nep based on the defect thickness and the defect length of the yarn Y in the first channel. By this configuration, the presence or absence of a nep can be accurately determined.

In the yarn monitoring device 8, the discrimination section 82 determines the presence or absence of mixing of a foreign substance, based on the defect brightness and the defect length of the yarn Y in the second channel. By this configuration, the presence or absence of mixing of a foreign substance can be accurately determined.

The yarn monitoring device 8 includes: a light emitting element configured to emit light toward a traveling region in which the yarn Y travels; a first light receiving element disposed so as to receive light that has been emitted from the light emitting element and part of which has been blocked by the yarn Y; and a second light receiving element disposed so as to receive light that has been emitted from the light emitting element and has been reflected by the yarn. The thickness is calculated based on the amount of light received by the first light receiving element, and the brightness is calculated based on the amount of light received by the second light receiving element. By this configuration, the thickness of the yarn Y and the brightness of the reflected light reflected by the yarn can be specifically calculated.

In the yarn monitoring device 8, the first defect-discrimination-target identification section identifies the first defect-discrimination-target portion based on the first variation signal and a first threshold, the second defect-discrimination-target identification section identifies the second defect-discrimination-target portion based on the second variation signal and a second threshold, and the discrimination section 82 identifies the first defect-discrimination-target portion and the second defect-discrimination-target portion overlapping each other in the longitudinal direction of the yarn Y as the portions corresponding to each other. By this configuration, the first defect-discrimination-target portion and the second defect-discrimination-target portion can be accurately identified.

In the yarnmonitoringdevice 8, the discrimination section 82 identifies a peak position of the first defect-discrimination-target portion. The discrimination section 82 identifies a peak position of the second defect-discrimination-target portion. The discrimination section 82 identifies, as the portions corresponding to each other, the first defect-discrimination-target portion and the second defect-discrimination-target portion the peak positions of which fall within a predetermined range in the longitudinal direction. By this configuration, the first defect-discrimination-target identification section and the second defect-discrimination-target identification section can be accurately identified.

The yarn monitoring device 8 includes the display 5b configured to display a discrimination result of the discrimination section 82 with a scatter diagram (see at least one of FIG. 10 to FIG. 12). By this configuration, the discrimination result of the discrimination section 82 can be effectively displayed by using the scatter diagram. The display 5b can display a kink so as to distinguish it from another yarn defect. The display 5b can display a foreign-substance mixed nep so as to distinguish it from another yarn defect.

The spinning machine 1 includes the yarn monitoring device 8, the drafting device 6, the air-j et spinning device 7, and the winding device 13. In the spinning machine 1 also, the yarn monitoring device 8 is included, and thus the above-described effect, that is, the effect of being able to accurately discriminate the yarn defect can be obtained.

In the yarn monitoring device 8, even a foreign-substance mixed nep that is not detected to be so thick in thickness detection of yarn Y by an optical method but is detected to be thick in thickness detection of the yarn Y by a capacitive method can be accurately discriminated as a yarn defect.

In the yarn monitoring device 8, a foreign-substance mixed nep can be accurately discriminated also by changing a setting of a clearing curve when determining the presence or absence of a nep in the first channel to stricter one. However, in this case, yarn Y is likely to be cut more frequently. In the yarn monitoring device 8, because a foreign-substance mixed nep can be discriminated in the third channel, without changing the setting of the clearing curve to stricter one (i.e., while preventing the yarn Y from being cut frequently), the foreign-substance mixed nep can be accurately discriminated. Thus, operation of the spinning machine 1 can be prevented from being hindered.

The first thickness criterion value, the second thickness criterion value, the first brightness criterion value, and the second brightness criterion value may be set to, for example, 10% more inside (lower) than a clearing limit for discrimination of a thickness defect such as a nep (the clearing curve CC used in the first channel) . The first thickness criterion value, the second thickness criterion value, the first brightness criterion value, and the second brightness criterion value may vary in the longitudinal direction such that the clearing limit for the thickness defect discrimination varies in the longitudinal direction in a manner similar to FIG. 5. Values of the clearing limit for kink discrimination and the clearing limit for foreign substance nep discrimination may be the same, or may be different. The values of the clearing limit for kink discrimination and the clearing limit for foreign substance nep discrimination may be the same as or may be different from the value of the clearing limit for thickness defect discrimination. When a first priority is given to discrimination of a defect type, there is no problem even if the values are the same. When the first priority is given to removal of a defect that cannot be caught completely by the thickness defect discrimination, it is desired that the values be different.

Although the embodiment according to the present invention and the modifications thereof have been described above, the present invention is not limited to the embodiment and the modifications. For example, the materials and shapes of the respective components are not limited to those described above, and various types of materials and shapes may be used. The present invention may be modified within the scope of the appended claims.

The discrimination section 82 according to the embodiment does not necessarily have to be incorporated in the unit controller 10, and may be incorporated in another controller that is separate from the unit controller 10 and can communicate with the device body 81, or may be incorporated in the device body 81 (in a case of the yarn monitoring device 8) . The display section configured to display a discrimination result of the discrimination section 82 is not limited to the display 5b, and may be a display screen provided to the device body 81, or may be another display.

In each spinning unit 2 according to the embodiment, the respective devices are disposed such that yarn Y supplied from the upper side is wound on the lower side in the machine height direction. However, the respective devices may be disposed such that yarn Y supplied from the lower side is wound on the upper side. In FIG. 1, the spinning machine 1 is illustrated in which each package P are wound in a cheese shape. However, the package P may be wound in a cone shape.

In the spinning unit 2 according to the embodiment, the yarn storage device 11 has a function of pulling out yarn Y from the air-j et spinning device 7. However, the yarn Y may be pulled out from the air-jet spinning device 7 by a delivery roller and a nip roller. When the yarn Y is pulled out from the air-jet spinning device 7 by a delivery roller and a nip roller, instead of the yarn storage device 11, a slack tube or a mechanical compensator, for example, configured to absorb slack of the yarn Y using suction airflow may be provided.

In the embodiment, the tension sensor 9 may be provided upstream of the yarn monitoring device 8 in the traveling direction of yarn Y. The unit controller 10 may be provided to each spinning unit 2. In the spinning unit 2, the waxing device 12 and the tension sensor 9 may be omitted.

In the embodiment, the defect thickness and the defect brightness are calculated based on the defect area and the defect length. However, the method of calculating the defect thickness and the defect brightness is not limited to this. For example, the peak thickness (the maximum value of the thickness) in a continuous portion in which the thickness is equal to or greater than a threshold may be simply used as the defect thickness. In the same manner, the peak brightness (the maximum value of the brightness) in a continuous portion in which the brightness is equal to or higher than a threshold may be simply used as the defect brightness. The respective thresholds described above are not limited to particular ones, and may be set or changed appropriately by the user. The respective thresholds may be different from each other, and at least some of them may be the same. Values for the second threshold described above may be different between the plus side and the minus side. Absolute values for the second threshold may be the same or may be different.

In the embodiment, the yarn monitoring device 8 according to the present invention is provided to the spinning machine 1 including the air-jet spinning device 7, which is what is called an air-jet spinning machine. However, the yarn monitoring device 8 maybe provided to an automatic winder or another yarn winding machine such as an open-end spinning machine. Herein, as described above, a yarn defect that is simply called "nep" means a nep into which no foreign substance is mixed. A yarn defect that falls under both "nep" and "mixing of a foreign substance" as described above means "foreign-substance mixed nep" .

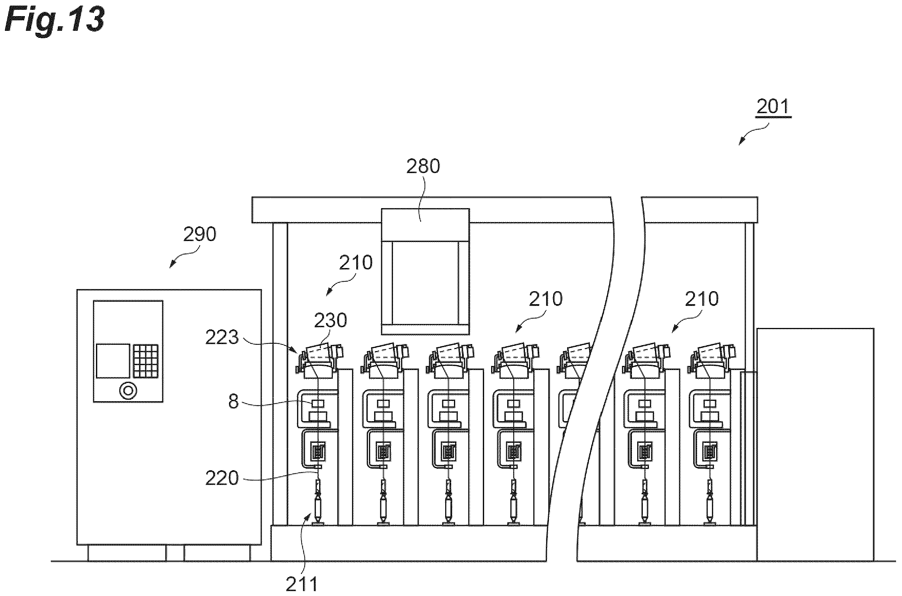

An automatic winder including the yarn monitoring device 8 according to the present invention is configured as follows, for example. As illustrated in FIG. 13, this automatic winder 201 includes a plurality of winder units 210 disposed in parallel, an automatic doffer 280, and a machine control device 290 as main components. Each winder unit 210 includes a yarn feeder 211 disposed so as to be able to feed yarn 220 and a winding device 223 configured to wind the yarn 220 fed from the yarn feeder 211 to form a package 230. The yarn monitoring device 8 is disposed between the yarn feeder 211 and the winding device 223. In the automatic winder 201 also, the yarn monitoring device 8 is included, and thus the above-described effect, that is, the effect of being able to accurately discriminate the yarn defect can be obtained.

## Claims

1. A yarn monitoring device (8) configured to monitor yarn traveling, the yarn monitoring device (8) comprising:
a discrimination section (82) configured to discriminate a type of a yarn defect of the yarn (Y), based on a combination of thickness of the yarn (Y) and brightness of reflected light that has been reflected by the yarn (Y) when the yarn (Y) has been irradiated with light, **characterized in that**
the discrimination section (82) includes a first defect-discrimination-target identification section and a second defect-discrimination-target identification section,
the first defect-discrimination-target identification section identifies, in a first variation signal for thickness, a first defect-discrimination-target portion for which a defect is to be assessed,
the second defect-discrimination-target identification section identifies, in a second variation signal for brightness, a second defect-discrimination-target portion for which a defect is to be assessed, and
the discrimination section (82) identifies the first defect-discrimination-target portion and the second defect-discrimination-target portion that correspond to each other in a longitudinal direction of the yarn (Y), and discriminates the type of the yarn defect based on the thickness of the first defect-discrimination-target portion and the brightness of the second defect-discrimination-target portion that have been identified as portions corresponding to each other, wherein the discrimination section (82) determines that a first yarn defect having warpage, tangle, or entanglement protruding in a direction intersecting a direction in which the yarn (Y) travels is present when the thickness of the first defect-discrimination-target portion is equal to or greater than a first thickness criterion value and the brightness of the second defect-discrimination-target portion is equal to or higher than a first brightness criterion value.

2. The yarn monitoring device (8) according to claim 1, wherein the second defect-discrimination-target portion includes a continuous portion in which the brightness is equal or higher than a threshold in the yarn (Y), the continuous portion coinciding with at least part of the first defect-discrimination-target portion.

3. The yarn monitoring device (8) according to claim 1, wherein the discrimination section (82) determines that a second yarn defect being a nep into which a foreign substance is mixed is present when the thickness of the first defect-discrimination-target portion is equal to or greater than a second thickness criterion value and the brightness of the second defect-discrimination-target portion is equal to or lower than a second brightness criterion value.

4. The yarn monitoring device (8) according to claim 3, wherein the second defect-discrimination-target portion includes a continuous portion in which the brightness is equal to or lower than a threshold in the yarn (Y), the continuous portion coinciding with at least part of the first defect-discrimination-target portion.

5. The yarn monitoring device (8) according to any one of claims 1 to 4, wherein the discrimination section (82) determines the presence or absence of a nep into which no foreign substance is mixed, based on the length of a continuous portion in which the thickness is equal to or greater than a threshold in the yarn (Y) and the thickness of the continuous portion.

6. The yarn monitoring device (8) according to any one of claims 1 to 5, wherein the discrimination section (82) determines the presence or absence of mixing of a foreign substance, based on the length of a continuous portion in which the brightness is equal to or lower than a threshold in the yarn (Y) and the brightness of the continuous portion.

7. The yarn monitoring device (8) according to any one of claims 1 to 6, further comprising:
a light emitting element (121) configured to emit light toward a traveling region in which the yarn (Y) travels;
a first light receiving element (131) disposed so as to receive light that has been emitted from the light emitting element (121) and part of which has been blocked by the yarn (Y); and
a second light receiving element (135) disposed so as to receive light that has been emitted from the light emitting element (121) and has been reflected by the yarn (Y), wherein
the thickness is calculated based on the amount of light received by the first light receiving element (131), and
the brightness is calculated based on the amount of light received by the second light receiving element (135).

8. The yarn monitoring device (8) according to any one of claims 1, 2, or 4, wherein
the first defect-discrimination-target identification section identifies the first defect-discrimination-target portion based on the first variation signal and a first threshold,
the second defect-discrimination-target identification section identifies the second defect-discrimination-target portion based on the second variation signal and a second threshold, and
the discrimination section (82) identifies the first defect-discrimination-target portion and the second defect-discrimination-target portion overlapping each other in the longitudinal direction of the yarn (Y) as portions corresponding to each other.

9. The yarn monitoring device (8) according to claim 8, wherein
the discrimination section (82) identifies a peak position of the first defect-discrimination-target portion,
the discrimination section (82) identifies a peak position of the second defect-discrimination-target portion, and
the discrimination section (82) identifies, as the portions corresponding to each other, the first defect-discrimination-target portion and the second defect-discrimination-target portion the peak positions of which fall within a predetermined range in the longitudinal direction.

10. The yarn monitoring device (8) according to any one of claims 1 to 9, further comprising a display section (5b) configured to display a discrimination result of the discrimination section (82) with a scatter diagram axes of which represent the thickness and the brightness.

11. The yarn monitoring device (8) according to claim 10, wherein the display section (5b) displays a first yarn defect having warpage, tangle, or entanglement protruding in a direction intersecting a direction in which the yarn (Y) travels so as to distinguish the first yarn defect from another yarn defect.

12. The yarn monitoring device (8) according to claim 11, wherein the display section (5b) displays a second yarn defect being a nep into which a foreign substance is mixed so as to distinguish the second yarn defect from another yarn defect.

13. A yarn winding machine (1) comprising:
the yarn monitoring device (8) as claimed in any one of claims 1 to 12;
a yarn feeder (6, 7) disposed so as to be able to feed the yarn (Y); and
a winding device (13) configured to wind the yarn (Y) fed from the yarn feeder (6,7) to form a package (P), wherein
the yarn monitoring device (8) is disposed between the yarn feeder (6, 7) and the winding device (13).

## Patentansprüche

1. Garnüberwachungsvorrichtung (8), die konfiguriert ist, um die Garnbewegung zu überwachen, wobei die Garnüberwachungsvorrichtung (8) Folgendes umfasst:
einen Unterscheidungsbereich (82), der konfiguriert ist, um einen Typ eines Garndefekts des Garns (Y) basierend auf einer Kombination der Dicke des Garns (Y) und der Helligkeit des reflektierten Lichts, das von dem Garn (Y) reflektiert wurde, als das Garn (Y) mit Licht bestrahlt worden ist, zu unterscheiden, **dadurch gekennzeichnet, dass**
der Unterscheidungsbereich (82) einen ersten Defekt-Unterscheidungs-Ziel-Identifizierungsbereich und einen zweiten Defekt-Unterscheidungs-Ziel-Identifizierungsbereich einschließt,
der erste Defekt-Unterscheidungs-Ziel-Identifizierungsbereich, in einem ersten Variationssignal für Dicke, einen ersten Defekt-Unterscheidungs-Ziel-Abschnitt identifiziert, für den ein Defekt zu bewerten ist,
der zweite Defekt-Unterscheidungs-Ziel-Identifizierungsbereich, in einem zweiten Variationssignal für Helligkeit, einen zweiten Defekt-Unterscheidungs-Ziel-Abschnitt identifiziert, für den ein Defekt zu bewerten ist, und
der Unterscheidungsabschnitt (82) den ersten Defekt-Unterscheidungs-Ziel-Abschnitt und den zweiten Defekt-Unterscheidungs-Ziel-Abschnitt identifiziert, die einander in einer Längsrichtung des Garns (Y) entsprechen, und den Typ des Garndefekts basierend auf der Dicke des ersten Defekt-Unterscheidungs-Ziel-Abschnitts und der Helligkeit des zweiten Defekt-Unterscheidungs-Ziel-Abschnitts, die als einander entsprechende Abschnitte identifiziert worden sind, unterscheidet, wobei der Unterscheidungsbereich (82) bestimmt, dass ein erster Garndefekt mit Verzug, Verknäuelung oder Verwicklung, der in einer Richtung herausragt, die eine Richtung kreuzt, in der sich das Garn (Y) bewegt, vorliegt, wenn die Dicke des ersten Defekt-Unterscheidungs-Ziel-Abschnitts gleich oder größer als ein erster Dickenkriterienwert ist und die Helligkeit des zweiten Defekt-Unterscheidungs-Ziel-Abschnitts gleich oder größer als ein erster Helligkeitskriterienwert ist.

2. Garnüberwachungsvorrichtung (8) nach Anspruch 1, wobei der zweite Defekt-Unterscheidungs-Ziel-Abschnitt einen kontinuierlichen Abschnitt einschließt, in dem die Helligkeit gleich oder höher als eine Schwelle im Garn (Y) ist, wobei der kontinuierliche Abschnitt mit zumindest einem Teil des ersten Defekt-Unterscheidungs-Ziel-Abschnitts übereinstimmt.

3. Garnüberwachungsvorrichtung (8) nach Anspruch 1, wobei der Unterscheidungsbereich (82) bestimmt, dass ein zweiter Garndefekt, der ein Nissen ist, in den ein Fremdstoff eingemischt ist, vorliegt, wenn die Dicke des ersten Defekt-Unterscheidungs-Ziel-Abschnitts gleich oder größer als ein zweiter Dickenkriterienwert ist und die Helligkeit des zweiten Defekt-Unterscheidungs-Ziel-Abschnitts gleich oder niedriger als ein zweiter Helligkeitskriterienwert ist.

4. Garnüberwachungsvorrichtung (8) nach Anspruch 3, wobei der zweite Defekt-Unterscheidungs-Ziel-Abschnitt einen kontinuierlichen Abschnitt einschließt, in dem die Helligkeit gleich oder niedriger als eine Schwelle im Garn (Y) ist, wobei der kontinuierliche Abschnitt mit zumindest einem Teil des ersten Defekt-Unterscheidungs-Ziel-Abschnitts übereinstimmt.

5. Garnüberwachungsvorrichtung (8) nach einem der Ansprüche 1 bis 4, wobei der Unterscheidungsabschnitt (82) basierend auf der Länge eines kontinuierlichen Abschnitts, in dem die Dicke gleich oder größer als eine Schwelle im Garn (Y) ist, und der Dicke des kontinuierlichen Abschnitts das Vorhandensein oder Nichtvorhandensein eines Nissens bestimmt, in das kein Fremdstoff eingemischt ist.

6. Garnüberwachungsvorrichtung (8) nach einem der Ansprüche 1 bis 5, wobei der Unterscheidungsabschnitt (82) basierend auf der Länge eines kontinuierlichen Abschnitts, in dem die Helligkeit gleich oder niedriger als eine Schwelle im Garn (Y) ist, und der Helligkeit des kontinuierlichen Abschnitts das Vorhandensein oder Nichtvorhandensein einer Einmischung einer Fremdsubstanz bestimmt.

7. Garnüberwachungsvorrichtung (8) nach einem der Ansprüche 1 bis 6, weiter umfassend:
ein lichtemittierendes Element (121), das zum Emittieren von Licht in Richtung eines Bewegungsbereichs konfiguriert ist, in dem sich das Garn (Y) bewegt;
ein erstes Lichtempfangselement (131), das so angeordnet ist, dass es Licht empfängt, das von dem lichtemittierenden Element (121) emittiert wurde und von dem ein Teil durch das Garn (Y) blockiert worden ist; und
ein zweites Lichtempfangselement (135), das so angeordnet ist, dass es Licht empfängt, das von dem lichtemittierenden Element (121) emittiert wurde und durch das Garn (Y) reflektiert worden ist, wobei
die Dicke basierend auf der durch das erste Lichtempfangselement (131) empfangenen Lichtmenge berechnet wird, und
die Helligkeit basierend auf der durch das zweite Lichtempfangselement (135) empfangenen Lichtmenge berechnet wird.

8. Garnüberwachungsvorrichtung (8) nach einem der Ansprüche 1, 2 oder 4, wobei
der erste Defekt-Unterscheidungs-Ziel-Identifizierungsbereich den ersten Defekt-Unterscheidungs-Ziel-Abschnitt basierend auf dem ersten Variationssignal und einer ersten Schwelle identifiziert,
der zweite Defekt-Unterscheidungs-Ziel-Identifizierungsbereich den zweiten Defekt-Unterscheidungs-Ziel-Abschnitt basierend auf dem zweiten Variationssignal und einer zweiten Schwelle identifiziert, und
der Unterscheidungsbereich (82) den ersten Defekt-Unterscheidungs-Ziel-Abschnitt und den zweiten Defekt-Unterscheidungs-Ziel-Abschnitt, die sich in der Längsrichtung des Garns (Y) überlappen, als einander entsprechende Abschnitte identifiziert.

9. Garnüberwachungsvorrichtung (8) nach Anspruch 8, wobei
der Unterscheidungsbereich (82) eine Spitzenposition des ersten Defekt-Unterscheidungs-Ziel-Abschnitts identifiziert,
der Unterscheidungsbereich (82) eine Spitzenposition des zweiten Defekt-Unterscheidungs-Ziel-Abschnitts identifiziert, und
der Unterscheidungsabschnitt (82), da die Abschnitte einander entsprechen, den ersten Defekt-Unterscheidungs-Ziel-Abschnitt und den zweiten Defekt-Unterscheidungs-Ziel-Abschnitt identifiziert, deren Spitzenpositionen innerhalb eines vorbestimmten Bereichs in der Längsrichtung fallen.

10. Garnüberwachungsvorrichtung (8) nach einem der Ansprüche 1 bis 9, weiter umfassend einen Anzeigebereich (5b), der konfiguriert ist, um ein Unterscheidungsergebnis des Unterscheidungsabschnitts (82) mit einem Streudiagramm anzuzeigen, dessen Achsen die Dicke und die Helligkeit darstellen.

11. Garnüberwachungsvorrichtung (8) nach Anspruch 10, wobei der Anzeigebereich (5b) einen ersten Garndefekt mit Verzug, Verknäuelung oder Verwicklung anzeigt, der in einer Richtung herausragt, die eine Richtung kreuzt, in der sich das Garn (Y) bewegt, sodass der erste Garndefekt von einem anderen Garndefekt unterschieden werden kann.

12. Garnüberwachungsvorrichtung (8) nach Anspruch 11, wobei der Anzeigebereich (5b) einen zweiten Garndefekt anzeigt, der eine Nisse ist, in die ein Fremdstoff eingemischt ist, sodass der zweite Garndefekt von einem anderen Garndefekt unterschieden werden kann.

13. Garnwickelmaschine (1), umfassend:
die Garnüberwachungsvorrichtung (8) nach einem der Ansprüche 1 bis 12;
eine Garnzuführung (6, 7), die so angeordnet ist, dass sie in der Lage ist, das Garn (Y) zuzuführen; und
eine Wickelvorrichtung (13), die konfiguriert ist, um das von der Garnzuführung (6, 7) zugeführte Garn (Y) zu wickeln, um ein Paket (P) zu bilden, wobei
die Garnüberwachungsvorrichtung (8) zwischen der Garnzuführung (6, 7) und der Wickelvorrichtung (13) angeordnet ist.

## Revendications

1. Dispositif de surveillance de fil (8) configuré pour surveiller un déplacement de fil, le dispositif de surveillance de fil (8) comprenant :
une section de discrimination (82) configurée pour discriminer un type d'un défaut de fil du fil (Y), sur la base d'une combinaison de l'épaisseur du fil (Y) et de la luminosité d'une lumière réfléchie qui a été réfléchie par le fil (Y) lorsque le fil (Y) a été exposé à une lumière, **caractérisé en ce que**
la section de discrimination (82) inclut une première section d'identification de cible de discrimination de défaut et une seconde section d'identification de cible de discrimination de défaut,
la première section d'identification de cible de discrimination de défaut identifie, dans un premier signal de variation pour l'épaisseur, une première portion cible de discrimination de défaut pour laquelle un défaut est à évaluer,
la seconde section d'identification de cible de discrimination de défaut identifie, dans un second signal de variation pour la luminosité, une seconde portion cible de discrimination de défaut pour laquelle un défaut est à évaluer, et
la section de discrimination (82) identifie la première portion cible de discrimination de défaut et la seconde portion cible de discrimination de défaut qui correspondent l'une à l'autre dans une direction longitudinale du fil (Y), et discrimine le type du défaut de fil sur la base de l'épaisseur de la première portion cible de discrimination de défaut et de la luminosité de la seconde portion cible de discrimination de défaut qui ont été identifiées en tant que portions correspondant l'une à l'autre, dans lequel la section de discrimination (82) détermine qu'un premier défaut de fil présentant une déformation, un enchevêtrement ou un emmêlement faisant saillie dans une direction croisant une direction dans laquelle le fil (Y) se déplace est présent lorsque l'épaisseur de la première portion cible de discrimination de défaut est supérieure ou égale à une première valeur critère d'épaisseur et la luminosité de la seconde portion cible de discrimination de défaut est supérieure ou égale à une première valeur critère de luminosité.

2. Dispositif de surveillance de fil (8) selon la revendication 1, dans lequel la seconde portion cible de discrimination de défaut inclut une portion continue dans laquelle la luminosité est supérieure ou égale à un seuil dans le fil (Y), la portion continue coïncidant avec au moins une partie de la première portion cible de discrimination de défaut.

3. Dispositif de surveillance de fil (8) selon la revendication 1, dans lequel la section de discrimination (82) détermine qu'un second défaut de fil étant un noeud, dans lequel un corps étranger est mêlé, est présent lorsque l'épaisseur de la première portion cible de discrimination de défaut est supérieure ou égale à une seconde valeur critère d'épaisseur et la luminosité de la seconde portion cible de discrimination de défaut est inférieure ou égale à une seconde valeur critère de luminosité.

4. Dispositif de surveillance de fil (8) selon la revendication 3, dans lequel la seconde portion cible de discrimination de défaut inclut une portion continue dans laquelle la luminosité est inférieure ou égale à un seuil dans le fil (Y), la portion continue coïncidant avec au moins une partie de la première portion cible de discrimination du défaut.

5. Dispositif de surveillance de fil (8) selon l'une quelconque des revendications 1 à 4, dans lequel la section de discrimination (82) détermine la présence ou l'absence d'un noeud dans lequel un corps étranger est mêlé, sur la base de la longueur d'une portion continue dans laquelle l'épaisseur est supérieure ou égale à un seuil dans le fil (Y) et de l'épaisseur de la portion continue.

6. Dispositif de surveillance de fil (8) selon l'une quelconque des revendications 1 à 5, dans lequel la section de discrimination (82) détermine la présence ou l'absence de mélange d'un corps étranger, sur la base de la longueur d'une portion continue dans laquelle la luminosité est inférieure ou égale à un seuil dans le fil (Y) et de la luminosité de la portion continue.

7. Dispositif de surveillance de fil (8) selon l'une quelconque des revendications 1 à 6, comprenant en outre :
un élément émetteur de lumière (121) configuré pour émettre une lumière vers une région de déplacement dans laquelle le fil (Y) se déplace ;
un premier élément récepteur de lumière (131) disposé de manière à recevoir une lumière qui a été émise depuis l'élément émetteur de lumière (121) et dont une partie a été bloquée par le fil (Y) ; et
un second élément récepteur de lumière (135) disposé de manière à recevoir une lumière qui a été émise depuis l'élément émetteur de lumière (121) et a été réfléchie par le fil (Y), dans lequel
l'épaisseur est calculée sur la base de la quantité de lumière reçue par le premier élément récepteur de lumière (131), et
la luminosité est calculée sur la base de la quantité de lumière reçue par le second élément récepteur de lumière (135).

8. Dispositif de surveillance de fil (8) selon l'une quelconque des revendications 1, 2 ou 4, dans lequel
la première section d'identification de cible de discrimination de défaut identifie la première portion cible de discrimination de défaut sur la base du premier signal de variation et d'un premier seuil,
la seconde section d'identification de cible de discrimination de défaut identifie la seconde portion cible de discrimination de défaut sur la base du second signal de variation et d'un second seuil, et
la section de discrimination (82) identifie la première portion cible de discrimination de défaut et la seconde portion cible de discrimination de défaut superposées l'une à l'autre dans la direction longitudinale du fil (Y) en tant que portions correspondant l'une à l'autre.

9. Dispositif de surveillance de fil (8) selon la revendication 8, dans lequel
la section de discrimination (82) identifie une position maximale de la première portion cible de discrimination de défaut,
la section de discrimination (82) identifie une position maximale de la seconde portion cible de discrimination de défaut, et
la section de discrimination (82) identifie, en tant que portions correspondant l'une à l'autre, la première portion cible de discrimination de défaut et la seconde portion cible de discrimination de défaut dont les positions maximales s'inscrivent dans une plage prédéterminée dans la direction longitudinale.

10. Dispositif de surveillance de fil (8) selon l'une quelconque des revendications 1 à 9, comprenant en outre une section d'affichage (5b) configurée pour afficher un résultat de discrimination de la section de discrimination (82) avec des axes de graphique en nuage de points qui représentent l'épaisseur et la luminosité.

11. Dispositif de surveillance de fil (8) selon la revendication 10, dans lequel la section d'affichage (5b) affiche un premier défaut de fil présentant une déformation, un enchevêtrement ou un emmêlement faisant saillie dans une direction croisant une direction dans laquelle le fil (Y) se déplace de manière à distinguer le premier défaut de fil d'un autre défaut de fil.

12. Dispositif de surveillance de fil (8) selon la revendication 11, dans lequel la section d'affichage (5b) affiche un second défaut de fil étant un noeud dans lequel un corps étranger est mêlé de manière à distinguer le second défaut de fil d'un autre défaut de fil.

13. Bobineuse de fils (1) comprenant :
le dispositif de surveillance de fil (8) selon l'une quelconque des revendications 1 à 12;
un passe-fil (6, 7) disposé de manière à pouvoir acheminer le fil (Y) ; et
un dispositif de bobinage (13) configuré pour bobiner le fil (Y) acheminé depuis le passe-fil (6, 7) pour former un enroulement (P), dans laquelle
le dispositif de surveillance de fil (8) est disposé entre le passe-fil (6, 7) et le dispositif de bobinage (13).
